Europäisches Patentamt

European Patent Office

Office européen des brevets

(11) Veröffentlichungsnummer: **0 219 753**
**B1**

(12) **EUROPÄISCHE PATENTSCHRIFT**

(45) Veröffentlichungstag der Patentschrift: **29.08.90**

(21) Anmeldenummer: **86113790.9**

(22) Anmeldetag: **04.10.86**

(51) Int. Cl.⁵: **A 61 K 7/00,** A 45 D 40/20,
B 43 K 19/00

(54) **Pudermine.**

(30) Priorität: **09.10.85 DE 3535999**

(43) Veröffentlichungstag der Anmeldung:
**29.04.87 Patentblatt 87/18**

(45) Bekanntmachung des Hinweises auf die
Patenterteilung:
**29.08.90 Patentblatt 90/35**

(84) Benannte Vertragsstaaten:
**DE FR GB IT**

(56) Entgegenhaltungen:
**DE-A-3 221 296**
**US-A-2 277 992**

(73) Patentinhaber: **Schwan-STABILO**
**Schwanhäusser GmbH & Co.**
**Maxfeldstrasse 3**
**D-8500 Nürnberg (DE)**

(72) Erfinder: **Brüchert, Werner, Dipl.-Ing. FH**
**Lenzstrasse 6**
**D-8507 Oberasbach (DE)**

(74) Vertreter: **LOUIS, PÖHLAU, LOHRENTZ &**
**SEGETH**
**Kesslerplatz 1 Postfach 3055**
**D-8500 Nürnberg-1 (DE)**

Die Akte enthält technische Angaben, die nach
dem Eingang der Anmeldung eingereicht
wurden und die nicht in dieser Patentschrift
enthalten sind.

Courier Press, Leamington Spa, England.

## Beschreibung

Die Erfindung betrifft eine Pudermine, die mit einer Schrumpffolie ummantelt ist.

Eine derartige Pudermine ist bspw. aus der DE—PS 32 21 296 bekannt. Nach dieser Druckschrift ist die Pudermine zylinderförmig mit senkrecht zur Zylinderachse stehenden Stirnflächen ausgebildet. Eine solche Pudermine wird dann bspw. mit einem Holzmantel verleimt und dann in den Handel gebracht. Der Verbraucher muß dann vor dem ersten Gebrauch die Pudermine zusammen mit dem Holzmantel anspitzen.

Ein Anspitzen der Pudermine und des Holzmantels im Herstellungswerk ist zwar möglich, aber beim Transport des Puderminenstiftes würde die Spitze abbrechen, sobald die Puderminen stärkeren Erschütterungen unterworfen sind.

Der Erfindung liegt nun die Aufgabe zugrunde, die Pudermine so auszubilden, daß sie vom Endverbraucher sofort verwendet werden kann, ohne daß die Gefahr besteht, daß sie beim Transport vom Herstellungswerk zum Endverbraucher abbricht.

Diese Aufgabe wird erfindungsgemäß dadurch gelöst, daß ein Ende der Pudermine als kegelförmige Spitze ausgebildet wird und daß die Schrumpffolienummantelung über einen Teil der Höhe der kegelförmigen Spitze fortgesetzt wird, wobei das Spitzenende selbst frei bleibt.

Es konnte überraschenderweise festgestellt werden, daß es zur Vermeidung eines Bruches der kegelförmigen Spitze ausreicht, diese nur teilweise mit der Schrumpffolie einzuschrumpfen, so daß sie sofort gebrauchsfähig ist. Es ist also wider Erwarten nicht notwendig, die kegelförmige Spitze vollständig einzuschrumpfen, um die Bruchsicherheit zu gewährleisten. Erst aufgrund dieser Erkenntnis war es überhaupt möglich, die gestellte Aufgabe zu lösen.

Versuche haben gezeigt, daß die Schrumpffolienummantelung sich zweckmäßigerweise über ca. zwei Drittel der Höhe kegelförmigen Spitze erstrecken sollte.

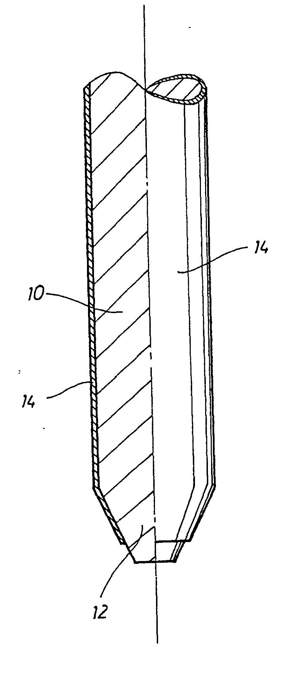

In der beiliegenden Zeichnung ist die Erfindung veranschaulicht. Dort ist mit 10 eine Pudermine bezeichnet, die eine kegelstumpfförmige Speitze 12 aufweist, Eine Schrumpffolienummantelung 15 ersteckt sich nicht nur über den zylindrischen Abschnitt der Pudermine, sondern auch über ca. zwei Drittel der Höhe der kegelförmigen Spitze der Pudermine.

## Patentansprüche

1. Pudermine, die mit einer Schrumpffolie (14) ummmantelt ist, dadurch gekennzeichnet, daß ein Ende der Pudermine (10) als kegelförmige Spitze zur Vermeidung eines Bruches (12) ausgebildet ist und daß sich die Schrumpffolienummantelung (14) über einen Teil der Höhe der kegelförmigen Spitze fortsetzt, wobei das Spitzenende selbst frei bleibt.

2. Pudermine nach Anspruch 1, dadurch gekennzeichnet, daß die Schrumpffolienummantelung (14) sich über ca. zwei Drittel der Höhe der kegelstumpfförmigen Spitze (12) erstreckt.

## Revendications

1. Mine pulvérulente enveloppée d'une feuille de contention 14, caractérisée en ce que l'extrémité de la mine 10 a la forme d'une pointe conique 12, et que pour éviter sa rupture, la gaine 14 se prolonge sur une portion de la hauteur de la pointe conique, de sorte que l'extrémité de la pointe demeure libre.

2. Mine pulvérulente selon la revendication 1, caractérisé en ce que la gaine 14 s'étend sur environ deux tiers de la hauteur de la pointe conique 12.

## Claims

1. Powder refill which is encased by a shrink film (14), characterized in that one end of the powder refill (10) is designed, in order to prevent breaking, as a conical tip (12) and in that the shrink-film casing (14) continues over a part of the height of the conical tip, the tip end itself remaining free.

2. Powder refill according to Claim 1, characterized in that the shrink-film casing (14) extends over approximately two thirds of the height of the frustoconical tip (12).